# EUROPEAN PATENT SPECIFICATION

(11) **EP 1 056 462 B1**
(45) Date of publication and mention of the grant of the patent: **25.07.2007**
(21) Application number: 99936031.6
(22) Date of filing: 24.02.1999
(51) Int. Cl.: A61K 8/60, A61Q 19/00

(54) **Use of rhamnolipids as cosmetics**
Verwendundung von Rhamnolipiden als Kosmetika
Utilisation de rhamnolipides comme agents cosmetiques

(30) Priority: 24.02.1998 US 75959 P
(43) Date of publication of application: 06.12.2000
(73) Proprietor: Paradigm Biomedical, Inc., New York, NY 10011-1101 (US)
(72) Inventor: PILJAC, Tatjana, Fremont, California 94555-3841 (US); PILJAC. Goran, Davis, California 95616 (US)
(74) Representative: Curtis, Philip Anthony
(86) International application number: PCT/US1999/003714
(87) International publication number: WO 1999/043334

(56) References cited:
- US-A- 4 902 512
- US-A- 5 455 232
- US-A- 5 514 661

## Description

The present invention relates to a cosmetic method for the treatment of wrinkles using rhamnolipids.

Typically, when an adult human receives an injury, either through burning of tissue or an incision in the skin tissue, the wound heals to leave a scar. This is even true in the case of post-surgical recovery where the wound has been closed with sutures (although scarring is generally less in such cases). This is not the case, however, for wounds to fetuses. It is known that wounds in fetuses heal rapidly and generally without scar formation until late in gestation. Reasons for this include:
1. *The dermis* is the location of the scar in adult wounds. As development progresses, dermal collagen is deposited and sulphated glycosaminoglycans (GAG) replace non-sulfated GAG of which hyaluronic acid (HA) is predominant.
*2. Fetal tissue* appears to be intrinsic in repair with reduction of fibrosis, and the maj or fetal cell type responsible for such repair may be the fetal fibroblast.
*3. The fetal immune system* is functionally immature relative to the adult immune system and plays a much less prominent role in fetal wound healing.
4. *The fetal extracellular matrix* (ECM) differs from that in adults in having HA, collagen, elastin, and adhesion glycoproteins as the major components.

It has been shown that hyaluronic acid levels in both fetal and adult sheep wounds rapidly increase until three days after wounding. This elevated level persists at least 21 days after wounding in the fetus, whereas it rapidly returns to baseline in the adult. In adult wounds, HA is deposited briefly within a fibrin and platelet plug. The HA is removed by hyaluronidase, and this provisional matrix is replaced by collagen and sulfated glycosaminoglycans. The deposition of collagen in fetal wounds is in a highly organized pattern that is indistinguishable from unwounded fetal dermis. Some of the major differences between fetal and adult repair are the temporal patterns of adhesion glycoproteins present in the wound, which are seen at the earliest stage of repair. These differences may lead to differences in cell mobility, migration, adhesion and proliferation.

*Cytokines.* Transforming growth factor-beta (TGF-beta) induces fibroplasia and increases wound tensile strength in adult wounds, and similar effects have been recorded in fetal wounds. In adults, activated macrophage products, such as cytokines and growth factors, progressively modify the local tissue environment, initially leading to destruction of tissue and later, i.e., in chronic delayed type hypersensitivity (DTH) reactions, causing replacement by connective tissue. The effects of macrophage-derived cytokines and growth factors occur in two phases. TNF, IL-1, and macrophage-derived chemokines acutely augment inflammatory reactions initiated by T-cells. These same cytokines also chronically stimulate fibroblast proliferation and collagen production. These slow actions of cytokines are augmented by the actions of macrophage-derived polypeptide growth factors. Platelet-derived growth factor, produced by activated macrophages, is a potent stimulator of fibroblast proliferation, whereas macrophage-derived growth factor (TGF- beta) augments collagen synthesis. Macrophage secretion of fibroblast growth factor causes endothelial cell migration and proliferation, leading to new blood vessel formation. The consequence of these slow actions of cytokines and growth factors is that prolonged activation of macrophages in a tissue, e. g., in the setting of chronic antigenic stimulation, leads to replacement of differentiated tissues by fibrous tissue. Fibrosis is the outcome of chronic DTH, when elimination of antigen and rapid resolution are unsuccessful.

There is thus a need to develop a cosmetic method for the treatment of wrinkles, the method comprising:
administering to a subject in need thereof, an effective amount of a composition comprising one or more rhamnolipids of Formula 1: 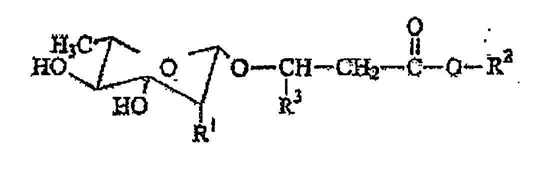 wherein
   R¹ = H, unsubstituted α-L-rhamnopyranosyl, α-L-rhamnopyranosyl substituted at the 2-position with a group of formula -O-C(=O)-CH=CH-R⁵, or -O-C(=O)-CH=H-R^{5;}
   R² = H, C1-C6 linear or branched alkyl, -CHR⁴-CH₂-COOH or -CHR⁴-CH₂-COOR⁶;
   R³ = -(CH₂)ₓ-CH₃, wherein x = 4-19;
   R⁴ = -(CH₂)_{y}-CH₃, wherein y =1-19;
   R⁵ = -(CH₂)_{z}-CH₃, wherein z =1-12; and
   R⁶ = C1-C6 linear or branched alkyl.

A more complete appreciation of the invention and many of the attendant advantages thereof will be readily obtained as the same becomes better understood by reference to the following detailed description.

The present invention relates to a cosmetic method for the treatment of wrinkles by administering cosmetic preparations and preparations and compositions comprising as the active ingredient, one or more rhamnolipids of Formula 1: 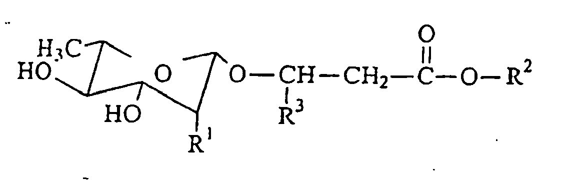
wherein R¹ = H, α-L-rhamnopyranosyl (either unsubstituted or substituted at the 2 position with a group of formula -O-C(=O)-CH=CH-R⁵), or -O-C(=O)-CH=CH-R⁵;
R² = H, C1-C6 linear or branched alkyl, preferably -CH₃),-CHR⁴-CH₂-COOH or -CHR⁴-CH₂-COOR⁶;
R³ = -(CH₂)ₓ-CH)₃, wherein x = 4-19;
R⁴ = -(CH₂)_{y}-CH)₃, wherein y = 1-19;
R⁵ = -(CH₂)₂-CH₃, wherein z = 1-12; and
R⁶ = C1-C6 linear or branched alkyl preferably -CH₃.

The rhamnolipids of the present invention can be prepared by conventional methods, preferably by fermentation, isolation and purification as described in U.S. Patent 5,455,232; 5,466,675 and 5,314,661, as well as BE 1005704A4, CA 2,129,542, JP 5-512946 and EP 93914523.1.

In the cosmetic methods of the present invention, rhamnolipid that is preferred has the structure of formula: 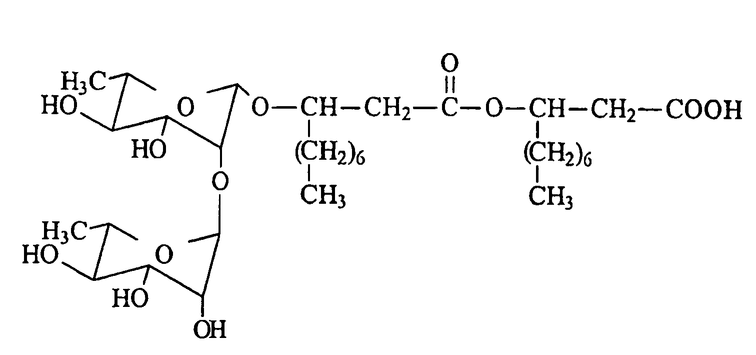 (α-L-rhamnopyranosyl-(1,2)-α-L-rhamnopyranosyl)-3-hyroxydecanoyl-3-hydroxydecanoic acid; hereafter referred to as "BAC-3")

Other preferred rhamnolipids include those wherein:
a) R¹ = -O-C(=O)-CH=CH-R⁵; R² = -CHR⁴-CH₂-COOH; R³ = -(CH₂)₆-CH₃; R⁴ = -(CH₂)₂H₃; and R⁵ = -(CH₂)₆-CH₃; or
b) R¹ = α-L-rhamnopyranosyl substituted at the 2-position by -O-C(=O)-CH=CH-R⁵; R² = -CHR⁴-CH₂-COOH; R³ = -(CH₂)₆-CH₃; R⁴ = -(CH₂)₆-CH₃; and R⁵ = -(CH₂)₆-CH₃; or
c) R¹ = -O-C(=O)-CH=CH-R⁵; R² = -CHR⁴-CH₂-COOCH₃; R³ = -(CH₂)₆-CH₃; R⁴ = -(CH₂)₂-CH₃; and R⁵ = -(CH₂)₆-CH₃; or
d) R¹ = α-L-rhamnopyranosyl substituted at the 2-position by -O-C(=O)-CH=CH-R⁵; R² = -CHR⁴-CH₂COOCH₃; R³ = -(CH₂)₆-CH₃; R⁴ = -(CH₂)₆-CH₃; and R⁵ = -(CH₂)₆-CH₃. The structures of a)-d) are shown below:

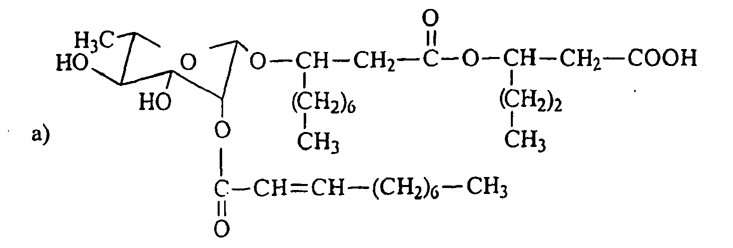

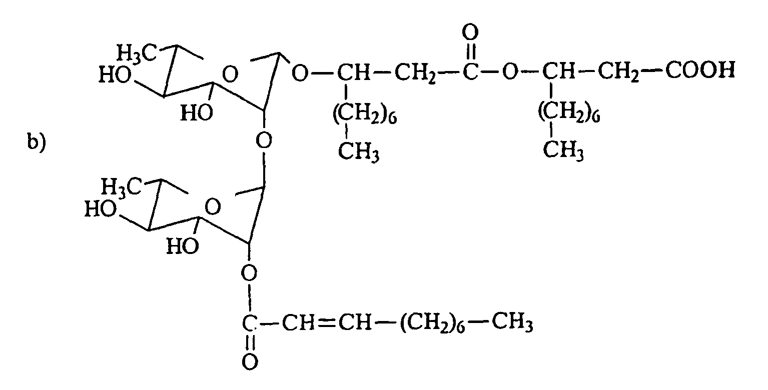

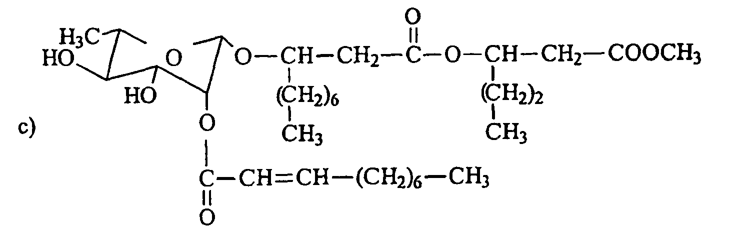

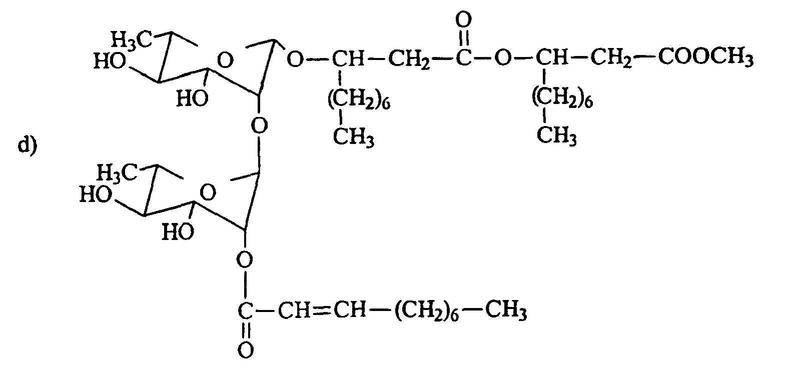

The toxicity and efficacy of these compounds can be further modified by varying the R groups as needed within the scope of Formula I.

It has now been found that these rhamnolipids are effective in re-epithelization of the skin.

Preferably, the rhamnolipid used in the cosmetic method is the BAC-3 rhamnolipid described above. The composition comprising the rhamnolipid can be in the form of neat liquid, suspension, dispersion, emulsion, cream, tincture, powder, ointment or lotion. Preferably, the composition is in an ointment. The amount of rhamnolipid used in the treatment is 0.001 % in the ointment up to 5% in the ointment, preferably from 0.01 to 1% in ointment, more preferably from 0.05 to 0.5% in ointment. (Unless otherwise indicated, all percentages are % by weight, based on total weight of the composition.) The ointment is applied directly to the subject area 1-5 times daily, preferably 2-3 times daily for a period of 1 day to 6 weeks.

The rhamnolipid containing composition used in the presently claimed method comprises one or more of the rhamnolipids in an amount effective to treat wrinkles as signs of aging. Such a cosmetic composition would be applied from 1 to 3 times per day to the affected area. The cosmetic composition could be in any of the topical forms noted above and contain similar amounts of rhamnolipid(s).

The composition comprising the one or more rhamnolipids can further include, if desired, one or more carriers and/or diluents conventionally used in the pharmaceutical and/or cosmetic industries.

## Claims

1. A, cosmetic method for the treatment of wrinkles, the method comprising:
administering to a subject in need thereof, an effective amount of a composition comprising one or more rhamnolipids of Formula 1: 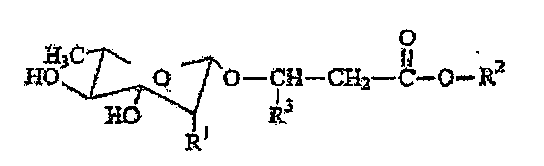
wherein R¹ = H, unsubstituted α-L-rhamnopyranosyl, α-L-rhamnopyranosyl substituted at the 2-position with a group of formula -O-C(=O)-CH=CH-R⁵, or -O-C(=O)-CH=CH-R⁵ ;
R² = H, C1-C6 linear or branched alkyl, -CHR⁴-CH₂-COOH or -CHR⁴-CH₂-COOR⁶;
R³ = -(CH₂)ₓ-CH₃, wherein x = 4-19;
R⁴ = -(CH₂)_{y}-CH₃, wherein y =1-19;
R⁵= -(CH₂)_{z}-CH₃, wherein z =1-12; and
R⁶= C1-C6 linear or branched alkyl.

2. A method according to claim 1, wherein said rhamnolipid of Formula 1 is α-L-rhamnopyranosyl-(1,2)-α-L-rhamnopyranosyl)-3-hydroxydecanoyl-3-hydroxydecanoic acid having the following formula:

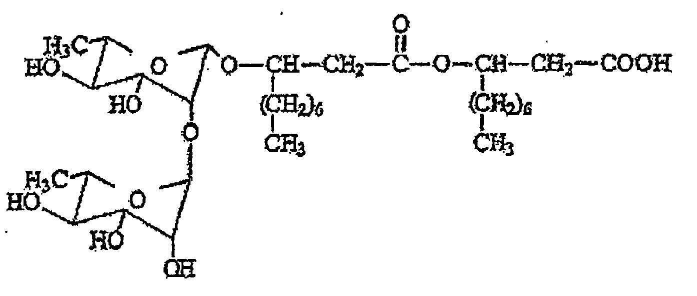

3. A method according to claim 1, wherein said one or more rhamnolipids of Formula 1 are selected from the group consisting of compounds of Formula 1 wherein:
R¹ = -O-C(=O)-CH=CH-R⁵, R² = -CHR⁴-CH₂-COOH, R³ = -(CH₂)₆-CH₃, R⁴ = -(CH₂)₂-CH₃, and R⁵ = -(CH₂)₆-CH₃;
R¹ = α-L-rhanmopyranosyl substituted at the 2-position by -O-C(=O)-CH-CH-R⁵, R² = -CHR⁴-CH₂-COOH, R³ = -(CH₂)₆-CH₃, R⁴ = -(CH₂)₆-CH₃, and R⁵ = -(CH₂)₆-CH_{3;}
R¹ = -O-C(=O)-CH=CH-R⁵, R² = -CHR⁴-CH₂-COOCH₃, R³ = -(CH₂)₆-CH₃, R⁴ = -(CH₂)₂-CH₃, and R⁵ = -(CH₂)₆CH₃; and
R¹ = α-L-rhamnopyranosyl substituted at the 2-position by -O-C(=O)-CH=CH-R⁵, R² = -CHR⁴-CH₂-COOCH₃, R³ = -(CH₂)₆-CH₃, R⁴ = -(CH₂)₆-CH₃, and R⁵ = -(CH₂)₆-CH₃₋

4. A method according to any preceding claim, wherein the composition is applied from 1 to 3 times per day.

5. A method according to any preceding claim, wherein the composition is in a topical form.

6. A method according to any preceding claim, wherein the composition further comprises one or more carriers and/or diluents.

## Patentansprüche

1. Kosmetisches Verfahren zur Behandlung von Falten, wobei das Verfahren Folgendes umfasst:
Verabreichen einer wirksamen Menge einer Zusammensetzung an eine bedürftige Person, die ein oder mehrere Rhamnolipide der Formel 1 umfasst: 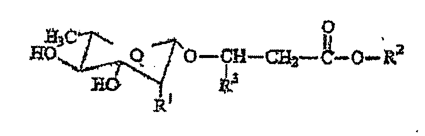
wobei R¹ = H, unsubstituiertes α-L-Rhamnopyranosyl, α-L-Rhamnopyranosyl, an der Position 2 substituiert mit einer Gruppe der Formel -O-C(=O)-CH=CH-R⁵ oder -O-C(=O)-CH=CH-R⁵:
R² = H, lineares oder verzweigtes C1-C6 Alkyl, -CHR⁴-CH₂-COOH oder -CHR⁴-CH₂-COOR⁶;
R³ = -(CH₂)ₓ-CH₃, wobei x = 4-19 ist;
R⁴= -(CH₂)_{y}-CH₃, wobei y = 1-19 ist;
R⁵ = -(CH₂)_{z}-CH₃, wobei z = 1-12 ist; und
R⁶ = lineares oder verzweigtes C1-C6 Alkyl ist.

2. Verfahren nach Anspruch 1, wobei das genannte Rhamnolipid der Formel 1 α-L-Rhamnopyranosyl-(1,2)-α-L-Rhamnopyranosyl)-3-hydroxydecanoyl-3-hydroxydecansäure mit der folgenden Formel:

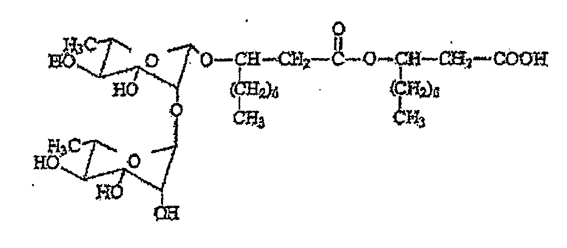

3. Verfahren nach Anspruch 1, wobei das/die genannte(n) eine oder mehreren Rhamnolipid(e) der Formel 1 ausgewählt ist/sind aus der Gruppe bestehend aus Verbindungen der Formel 1, wobei:
R¹ = -O-C(=O)-CH=CH-R⁵, R² = -CHR⁴-CH₂-COOH, R³ = -(CH₂)₆-CH₃, R⁴= - (CH₂)₂-CH₃, und R⁵=(CH₂)₆-CH₃;
R¹ = α-L-Rhamnopyranosyl, substituiert an der Position 2 durch -O-C(=O)-CH=CH-R⁵, R² = -CHR⁴-CH₂-COOH, R³ = -(CH₂)₆-CH₃, R⁴ = -(CH₂)₆-CH₃, und R⁵= -(CH₂)₆-CH₃;
R¹ = -O-C(=O)-CH=CH-R⁵, R² = -CHR⁴-CH₂-COOCH₃, R³ = -(CH₂)₆-CH₃, R⁴ = -(CH₂)₂-CH₃, und R⁵ = -(CH₂)₆-CH₃; und
R¹ = α-L-Rhamnopyranosyl, substituiert an der Position 2 durch -O-C(=O)-CH=CH-R⁵, R² = -CHR⁴-CH₂-COOCH₃, R³ = -(CH₂)₆-CH₃, R⁴ = -(CH₂)₆-CH₃, und R⁵= -(CH₂)₆-CH₃.

4. Verfahren nach einem der vorherigen Ansprüche, wobei die Zusammensetzung 1 bis 3 Mal täglich angewendet wird.

5. Verfahren nach einem der vorherigen Ansprüche, wobei die Zusammensetzung in topischer Form vorliegt.

6. Verfahren nach einem der vorherigen Ansprüche, wobei die Zusammensetzung ferner einen oder mehrere Träger und/oder Verdünnungsmittel umfasst.

## Revendications

1. Procédé cosmétique pour le traitement des rides, comprenant :
l'administration à un sujet qui en a besoin d'une quantité efficace d'une composition comprenant un ou plusieurs rhamnolipides de la Formule 1 : 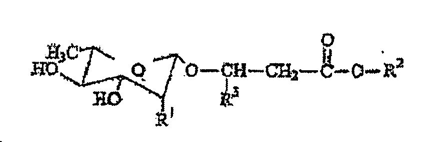
où R¹ = H, α-L-rhamnopyranosyle non substitué, α-L-rhamnopyranosyle substitué à la position 2 avec un groupe de la formule -O-C(=O)-CH=CH-R⁵, ou -O-C(=O)-CH=CH-R⁵ :
R² = H, alkyle C1-C6 linéaire ou ramifié, -CHR⁴-CH₂-COOH ou -CHR⁴-CH₂-COOR⁶ ;
R³ = -(CH2)ₓ-CH₃, où x = 4-19 ;
R⁴ = - (CH₂) _{y}-CH₃, où y = 1-19 ;
R⁵ = - (CH₂)_{z}-CH₃, où z = 1-12 ; et
R⁶ = alkyle C1 - C6 linéaire ou ramifié

2. Procédé selon la revendication 1, dans lequel ledit rhamnolipide de la Formule 1 est l'acide α-L-rhamnopyranosyle-(1,2)-α-L-rhamnopyranosyle)-3-hydroxydécanoyle-3-hydroxydécanoique ayant la formule suivante :

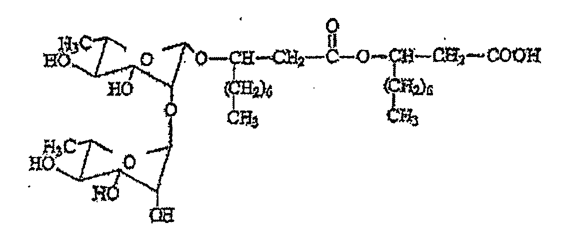

3. Procédé selon la revendication 1, dans lequel un ou plusieurs rhamnolipides de la Formule 1 sont sélectionnés dans le groupe comprenant des composés de la Formule 1, où
R¹ = -O-C(=O)-CH=CH-R⁵, R²= -CHR⁴-CH₂-COOH, R³= -(CH₂)₆ = CH₃, R⁴ = -(CH₂)₂-CH₃, et R⁵ = -(CH₂)₆-CH₃
R¹ = α-L-rhamnopyranosyle substitué à la position 2 par -O-C(=O)-CH=CH-R⁵, R² = -CHR⁴-CH₂-COOH, R³ = -(CH₂)₆ = CH₃, R⁴ = -(CH₂)₆-CH₃, et R⁵ = -(CH₂)₆-CH₃;
R¹ = -O-C(=O)-CH=CH-R⁵, R² = -CHR⁴-CH₂-COOCH₃, R³ =-(CH₂)₆ - CH₃, R⁴ = -(CH₂)₂-CH₃, et R⁵= - (CH₂)₆-CH₃; et
R¹ = α-L-rhamnopyranosyle substitué à la position 2 par -O-C(=O)-CH=CH-R⁵, R² = -CHR⁴-CH₂-COOH₃, R³ = -(CH₂)₆-CH₃, R⁴ = -(CH₂)₆-CH₃, et R⁵ = -(CH₂)₆-CH₃.

4. Procédé selon l'une quelconque des revendications précédentes, dans lequel la composition est appliquée 1 à 3 fois par jour.

5. Procédé selon l'une quelconque des revendications précédentes, dans lequel la composition est de forme topique.

6. Procédé selon l'une quelconque des revendications précédentes, dans lequel la composition comprend en outre un ou plusieurs excipients et/ou diluants.
